# EUROPÄISCHE PATENTSCHRIFT

(11) **EP 3 157 426 B1**
(45) Veröffentlichungstag und Bekanntmachung des Hinweises auf die Patenterteilung: **20.06.2018**
(21) Anmeldenummer: 15723134.1
(22) Anmeldetag: 27.03.2015
(51) Int. Cl.: A61B 5/00, A61B 5/11, A61B 5/18, A61B 3/00, A61B 3/14, G08B 21/06, B60W 40/08

(54) **VORRICHTUNG, VERFAHREN UND COMPUTERPROGRAMM ZUR DETEKTION EINES SEKUNDENSCHLAFS**
DEVICE, METHOD, AND COMPUTER PROGRAM FOR DETECTING MICRO- SLEEP
DISPOSITIF, PROCÉDÉ ET PRODUIT-PROGRAMME INFORMATIQUE PERMETTANT DE DÉTECTER UN MICRO-SOMMEIL

(30) Priorität: 20.06.2014 DE 102014211898
(43) Veröffentlichungstag der Anmeldung: 26.04.2017
(73) Patentinhaber: FRAUNHOFER-GESELLSCHAFT zur Förderung der angewandten Forschung e.V., 80636 München (DE); Technische Universität Ilmenau, 98693 Ilmenau (DE)
(72) Erfinder: KRENZER, Daniel, 99848 Wutha-Farnroda (DE); HESS, Albrecht, 98667 Schönbrunn (DE); KÁTAI, András, 98693 Ilmenau (DE); PAULIGK, Matthias, 98693 Ilmenau (DE); FRITZSCHE, Paul, 98693 Ilmenau (DE); TILGNER, Joachim, 63456 Hanau (DE); HÄNSEL, Michael, 98693 Ilmenau (DE); CHILIAN, Anja, 98693 Ilmenau (DE); HARCZOS, Tamás, 98704 Wolfsberg Ortsteil Wümbach (DE); KÜNZEL, Johannes-Wolf, 10315 Berlin (DE); HUSAR, Peter, 98693 Ilmenau (DE); WIEDE, Christian, 09126 Chemnitz (DE)
(74) Vertreter: Stöckeler, Ferdinand
(86) Internationale Anmeldenummer: PCT/EP2015/056748
(87) Internationale Veröffentlichungsnummer: WO 2015/192980

(56) Entgegenhaltungen:
- EP-A1- 2 330 582
- DE-A1- 19 715 519
- JP-A- 2011 043 961
- US-A1- 2005 073 136
- US-A1- 2008 101 659
- US-A1- 2011 077 548
- US-A1- 2011 216 181
- US-A1- 2012 179 008

## Beschreibung

Die vorliegende Erfindung bezieht sich auf eine Vorrichtung, ein Verfahren und ein Computerprogramm zur Detektion eines Sekundenschlafs. Ausführungsbeispiele zeigen einen Sekundenschlafwarner.

Fahrerassistenzsysteme, die auch Sekundenschlafwarner umfassen, können bereits in einigen Serienfahrzeugen vorgefunden werden. Diese basieren auf einer statistischen Auswertung von beispielsweise am CAN-Bus verfügbaren Daten (z. B. Lenkwinkel, Gas- und Bremsverhalten, usw.). Anders ausgedrückt werten bisher in Serienfahrzeugen erhältliche Fahrerassistenzsysteme Daten aus, die nur indirekt und unpräzise Rückschlüsse über den Zustand des Fahrers und insbesondere auf den Sekundenschlaf zulassen.

Ferner ist es möglich, Nachrüstsysteme direkt an der Person zu befestigen. Beispielhafte Befestigungsorte sind das Ohr oder die Hand. So kann das System z. B. auf eine Kopfneigung oder auf den Hautleitwert reagieren, um daraus den Sekundenschlaf zu erkennen. Diese Systeme haben wie die o. g. Fahrerassistenzsysteme den Nachteil, dass Daten ausgewertet werden, die nur indirekte und unpräzise Rückschlüsse über den Zustand des Fahrers und insbesondere auf den Sekundenschlaf zulassen. Ferner sind diese Systeme für den Nutzer aufwendig in der Handhabung, unangenehm zu Tragen und werden von den potenziellen Nutzern kaum akzeptiert.

Darüber hinaus ist es möglich, eine Brille mit integrierten Kameras, die auf die Augen ausgerichtet sind, einzusetzen, die videobasiert den Sekundenschlaf über den Lidschluss erkennen können. Jedoch sind auch diese Systeme für den Nutzer aufwendig in der Handhabung, unangenehm zu Tragen und werden ebenfalls von den potenziellen Nutzern kaum akzeptiert.

Weitere bekannte, kamerabasierte Sekundenschlafwarner nutzen beispielsweise voreingestellte Schwellwerte zur Lidschlussbestimmung. Diese sind jedoch wenig zuverlässig, da voreingestellte Referenzwerte für die Sekundenschlafbestimmung verwendet werden.

Das Dokument US 2011/216181 A1 offenbart einen Sekundenschlafdetektor, bei dem anhand einer Videoaufnahme ein Lidschluss auf der Grundlage eines individuell anpassbaren Schwellwertes detektiert wird. Es wird ebenfalls die Dauer eines Lidschlusses bestimmt, wofür aber kein individuell anpassbarer Schwellwert vorgesehen ist.

Der vorliegenden Erfindung liegt somit die Aufgabe zugrunde, ein verbessertes Konzept für die Warnung bei Sekundenschlaf zu schaffen.

Diese Aufgabe wird durch den Gegenstand der unabhängigen Patentansprüche gelöst. Erfindungsgemäße Weiterbildungen sind in den Unteransprüchen definiert.

Ausführungsbeispiele zeigen eine Vorrichtung zur Detektion eines Sekundenschlafs. Die Vorrichtung weist eine Videoaufnahmevorrichtung zum videobasierten Beobachten einer Person sowie einer Augenpartie der Person auf, wobei die Videoaufnahmevorrichtung ausgebildet ist, eine Bildfolge der Person und der Augenpartie aufzunehmen und an eine Schwellwertermittlungsvorrichtung auszugeben. Die Schwellwertermittlungsvorrichtung ist ausgebildet, um einen oder mehrere individuell an die Person angepasste Schwellwerte aus der Bildfolge abzuleiten. Ferner umfasst die Vorrichtung einen Schwellwertauswerter, der ausgebildet ist, um basierend auf dem einen oder mehreren individuell angepassten Schwellwerten zu entscheiden, ob der Sekundenschlaf der Person eingetreten ist, wobei der eine oder mehrere individuell angepassten Schwellwerte genutzt werden, um innerhalb des tatsächlichen zeitlichen Verlaufs der Augenöffnung ein Passieren der Schwellwerte und damit das Vorliegen des Sekundenschlafes festzustellen. Passieren kann das Erreichen sowie das Überschreiten bzw. Unterschreiten eines Schwellwertes bedeuten. So ist es beispielsweise sinnvoll, ein Erreichen oder Unterschreiten des ersten Schwellwertes (Lidschlussgrad) und ein Erreichen oder Überschreiten des zweiten Schwellwertes (Dauer des Unterschreitens des ersten Schwellwertes, z. B. Dauer, die die Lider als geschlossen erkannt werden) zu bestimmen. Eine Verarbeitung der Bildfolge kann ferner in Echtzeit erfolgen.

Der Erfindung liegt die Erkenntnis zugrunde, dass eine insbesondere in Bezug auf die Person kontaktfreie Detektion eines Sekundenschlafs mittels direkter Messung des Augenlidschlusses möglich ist, welches ein guter Indikator für einen eingetretenen Sekundenschlaf ist. Der Lidschluss (bzw. die Augenöffnung) wird in diesem videobasierten Vorgang mittels eines vordefinierten Modells ermittelt, wobei das Modell individuell auf die Person angepasst wird. Hierzu könnte ein Basis-Kopfmodell, individuell an die zu beobachtende Person angepasst, zum Einsatz kommen. Die Messung des Augenlidschlusses als direktes Merkmal für einen Sekundenschlaf ist der Messung von indirekten Merkmalen bereits dadurch überlegen, dass keine aufwendigen und dadurch fehleranfälligen Algorithmen auf indirekte Merkmale, wie beispielsweise die Kopfneigung oder den Hautleitwert, angewendet werden brauchen. Ferner ist eine kontaktlose Messung einer kontaktbehafteten Messung in Bezug auf den Komfort und die Akzeptanz deutlich überlegen. Eine beispielsweise in einem Auto montierte Vorrichtung benötigt keine weitere Anpassung (bspw. eine Kalibrierung durch den Nutzer), sodass eine beobachtete Person gar nicht merkt, dass sie von Kameras aufgenommen wird. Dies ist z. B. bei einer speziellen Brille nicht gegeben. Diese muss vor Fahrtantritt aufgesetzt werden und vermittelt der Person dauerhaft ein anderes Gefühl während der gesamten Tragedauer. Dieser psychologische Effekt trägt wesentlich zum Wohlbefinden des Nutzers bei.

Die Kriterien bzw. Schwellwerte, die genutzt werden um aus dem beobachteten Lidschlussverlauf den Sekundenschlaf zu detektieren (bzw. das Modell individuell an die Person anzupassen), können aus der Beobachtung der Person heraus bestimmt und so individuell an die Person angepasst werden. Der Vorteil dabei ist, dass die Anatomie und Physiologie der Person einbezogen wird und daher wesentlich besser der Sekundenschlaf detektiert werden kann als mit allgemeinen Standardwerten für die Kriterien bzw. Schwellwerte. Individuell an die Anatomie und Physiologie der Person angepasste Kriterien haben eine verbesserte Aussagekraft bzgl. der einzelnen Person gegenüber den allgemeinen Standardwerte, die z. B. statistisch, über größere Personengruppen hinweg ermittelt werden. Diese verbesserte Aussagekraft erhöht die Verlässlichkeit der individuellen Warnungen bei Sekundenschlaf signifikant. Die Erfindung zeigt einen ersten individuell angepassten Schwellwert, der ein Lidschluss eines Auges ist, wobei der Lidschluss zwischen einem ersten individuell ermittelten Referenzwert, der ein geöffnetes Auge beschreibt und einem zweiten individuell ermittelten Referenzwert, der ein geschlossenes Auge beschreibt, liegt, und der Schwellwert den Übergang des geöffneten zum geschlossenen Auge kennzeichnet, wobei sich ein zweiter individuell ermittelter Schwellwert auf eine zeitliche Dauer des Lidschlusses bezieht. Zur Erfindung gehört ferner ein Schwellwertauswerter, der ausgebildet ist, ein Passieren des ersten Schwellwerts zu detektieren sowie eine Dauer des Passierens zu bestimmen, wobei der Sekundenschlaf festgestellt wird, wenn die Dauer den zweiten Schwellwert passiert. Der Sekundenschlaf wird demnach erkannt, wenn der erste Schwellwert (z. B. unter Beachtung einer Hysterese) unterschritten oder erreicht ist, womit geschlossene Augenlider erkannt wurden, und die Dauer, die die Lider geschlossen sind, den zweiten Schwellwert erreicht oder übertritt.

Weitere individuell angepasste Schwellwerte können zu weiteren Parametern bzw. Qualitätsmaßen ermittelt werden und als Kriterien für das Vorliegen des Sekundenschlafes in die Auswertung der individuell angepassten Schwellwerte mit einbezogen werden. Die Nutzung solcher Schwellwerte kann z.B. die Erkennung des Sekundenschlafs erleichtern, indem Parameter wie z.B. eine Position, eine Ausrichtung oder eine Größe von markanten Mustern oder Punkten bestimmt wird. Die markanten Muster oder Punkte können z. B. der Kopf, das Gesicht, die Augen, die Iris, die Pupille oder der Pupillenmittelpunkt der Person sein. Die weiteren Beispiele für individuell angepasste Schwellwerte können einzeln oder in beliebiger Kombination, beispielsweise auch mit den vorher genannten individuell angepassten Schwellwerte (Lidschlussgrad und Dauer des Lidschlusses) verwendet werden. Ebenso können sie die vorher genannten individuell angepassten Schwellwerte (Lidschlussgrad und Dauer des Lidschlusses) ersetzen.

Gemäß weiteren Ausführungsbeispielen ist die Schwellwertermittlungsvorrichtung ausgebildet, die individuell ermittelten Referenzwerte und einen ersten oder mehrere individuell ermittelte Schwellwerte kontinuierlich durch die Auswertung auftretender Lidschläge während des Betriebs anzupassen. Dies ist vorteilhaft, da sich die Lidschlussdauer, z. B. bei einem Zwinkern, während der Beobachtungsdauer, beispielsweise durch Ermüdung oder einsetzende Dämmerung, verändern kann. Daher kann die Schwellwertermittlungsvorrichtung ein Zwinkern bzw. einen gewollten oder ungewollten spontanen Lidschlag anhand eines Musters im zeitlichen Augenöffnungsverlauf bestimmen, wobei das Muster, aus Richtung des ersten Referenzwertes ein Unterschreiten des Schwellwertes und eine Rückkehr in Richtung des ersten Referenzwertes innerhalb einer vorgegebenen Zeit aufweist.

Ausführungsbeispiele zeigen ferner die Schwellwertermittlungsvorrichtung, die ausgebildet ist, den einen oder mehrere individuell angepasste Schwellwerte an die Anatomie und Physiologie des Fahrers anzupassen. So können z. B. für jede Person individuell ermittelte Schwellwerte für die Sekundenschlafbestimmung herangezogen werden. Solange die Vorrichtung nach dem Einschalten noch keine individuellen Referenz- oder Schwellwerte für die Person ermittelt hat, können auch vordefinierte allgemeinere Werte genutzt werden, damit die Vorrichtung trotzdem in dieser Zeit schon arbeiten kann.

Ferner kann der erste individuell angepasste Schwellwert eine Hysterese aufweisen, durch dieselbe ein Lidschluss bei einem geringeren Augenöffnungswinkel beim Übergang vom geöffneten zum geschlossenen Auge und bei einem größeren Augenöffnungswinkel beim Übergang vom geschlossenen zum geöffneten Auge festgelegt wird.

Weitere Ausführungsbeispiele zeigen die Schwellwertermittlungsvorrichtung, die eine Kopfpose ermittelt und wobei die individuell angepassten Schwellwerte anhand der ermittelten Kopfpose angepasst werden. Dies kann vorteilhaft eingesetzt werden, um den kontinuierlich ermittelten Augenöffnungsverlauf für unterschiedliche Kopfposen quantitativ vergleichbar zu machen, denn es ändert sich bei einem Drehen des Kopfes die für die Vorrichtung sichtbare Fläche des Auges, die daher z. B. beim Kippen des Kopfes nach vorne kleiner erscheint. Ebenso kann die Schwellwertermittlungsvorrichtung ausgebildet sein, auf Basis der Kopfpose den Lidschluss anhand beider Augen der Person zu bestimmen, z. B. wenn beide Augen für die Vorrichtung gut sichtbar sind, oder anhand eines Auges der Person zu bestimmen, wenn beispielsweise nur ein Auge für die Vorrichtung sichtbar ist.

Ausführungsbeispiele zeigen ferner, dass der zweite individuell angepasste Schwellwert eine längere Dauer als die Dauer eines Zwinkerns angibt. Ferner kann der zweite individuell angepasste Schwellwert an die aktuelle Geschwindigkeit, mit der sich die Person, z. B. mit einem Fahrzeug, fortbewegt, angepasst sein. Zur Bestimmung der aktuellen Geschwindigkeit, mit der sich die Person fortbewegt kann die Vorrichtung z. B. über einen GPS-Empfänger und/oder einen Beschleunigungssensor verfügen, der ausgebildet ist, eine Fortbewegungsgeschwindigkeit der Vorrichtung zu erfassen, wobei davon ausgehend auf die Geschwindigkeit der Person bzw. des Fahrzeuges geschlossen werden kann. Die Schwellwertermittlungsvorrichtung 20 ist ferner ausgebildet, den einen oder mehrere individuell angepasste Schwellwerte 80 und 85 an die Fortbewegungsgeschwindigkeit anzupassen. Dies ermöglicht eine Abschaltung der Sekundenschlaferkennung bzw. der Warnung bei Sekundenschlaf wenn sich die Person z. B. in einem Fahrzeug befindet, dieses sich aber nicht fortbewegt. In einem weiteren Ausführungsbeispiel kann die Vorrichtung die Fortbewegungsgeschwindigkeit auch als Eingangssignal empfangen werden.

Gemäß weiteren Ausführungsbeispielen weist die Vorrichtung eine Beleuchtungsquelle auf, die ausgebildet ist, Strahlung oberhalb eines für die Person sichtbaren Wellenlängenbereichs auszusenden, wobei die Videoaufnahmevorrichtung ausgebildet ist, die Strahlung zu detektieren. Dies ist vorteilhaft, um die Person (auch bei sich ändernden äußeren Lichtverhältnissen) gleichmäßig zu beleuchten, ohne dass die Person die Beleuchtung registriert und durch dieselbe gestört wird. Ferner kann die Vorrichtung eine weitere Strahlenquelle aufweisen, die von der Strahlenquelle entfernt angeordnet ist, wobei die Videoaufnahmevorrichtung ausgebildet ist, eine Kombination von Bildaufnahmen, bei denen die Person durch aufeinanderfolgendes Ausleuchten der Augenpartie mit der Strahlenquelle und der weiteren Strahlenquelle beleuchtet ist, zu erzeugen, um Reflexe im Bereich der Augenpartie der Person zu vermeiden oder zu vermindern. Vorteilhaft an dieser Anordnung kann die Verbesserung der Bildqualität und damit herrührend die Genauigkeit der Sekundenschlafbestimmung sein, da ohne die Reflexionsvermeidung bzw. - verminderung beispielsweise an Brillen oder einer Pupille auftretende Reflexionen ein oder beide Augen der Person überdecken können und eine Sekundenschlafbestimmung so erschwert wird.

Ferner zeigen Ausführungsbeispiele ein Verfahren zur Detektion eines Sekundenschlafs, das als Computerprogramm implementiert sein kann.

Bevorzugte Ausführungsbeispiele der vorliegenden Anmeldung werden nachfolgend Bezug nehmend auf die beiliegenden Zeichnungen näher erläutert. Es zeigen:
- Fig. 1: ein schematisches Blockdiagramm einer Vorrichtung zur Detektion eines Sekundenschlafs;
- Fig. 2: eine schematische Darstellung eines Verlaufs der Augenöffnung mit individuell ermittelten Referenzwerten sowie individuell angepassten Schwellwerten zur Detektion des Sekundenschlafs;
- Fig. 3: ein schematisches Blockdiagramm einer Vorrichtung zur Detektion eines Sekundenschlafs in einer von Fig. 1 abweichenden Darstellung, die detailliertere Funktionsblöcke aufweist;
- Fig. 4: eine schematische Darstellung einer auf eine Person ausgerichteten Videoaufnahmevorrichtung;
- Fig. 5: eine schematische Darstellung eines Fahrzeuginnenraums mit beispielhaften Anordnungen der Vorrichtung zur Detektion eines Sekundenschlafs; und
- Fig. 6: ein schematisches Flussdiagramm eines Verfahrens zur Detektion eines Sekundenschlafs.

In der nachfolgenden Beschreibung der Figuren werden gleiche oder gleichwirkende Elemente mit den gleichen Bezugszeichen versehen, so dass deren Beschreibung in den unterschiedlichen Ausführungsbeispielen untereinander austauschbar ist.

Übermüdung von Personen (z. B. Fahrern eines Fahrzeuges) kann zu sinkender Aufmerksamkeit und eintretendem Sekundenschlaf führen. Sekundenschlaf wird hier als Synonym für einen kurzen, eintretenden Schlaf genutzt und schließt beispielsweise auch den Mikroschlaf ein. Hierdurch werden ca. 25% der Unfälle mit Todesfolge auf deutschen Autobahnen verursacht. Derzeit ist kein System auf dem Markt bekannt, welches zuverlässig bei Sekundenschlaf warnt. Daher wird im Folgenden eine Vorrichtung zur Detektion eines Sekundenschlafs vorgestellt, die präzise und zuverlässig den Lidschluss einer Person detektiert um daraus den Sekundenschlaf der Person abzuleiten, Sekundenschlaf bei einer Person detektiert, eine Warnung beim Auftreten von Sekundenschlaf ausgibt und eine einfache Nutzbarkeit des Systems zur Sekundenschlafwarnung ermöglicht.

Das Gesamtsystem kann aus einem Kamerabild, in dem das Gesicht der zu überwachenden Person abgebildet ist, individuell angepasste Schwellwerte zur Detektion des Sekundenschlafs der Person ableiten und warnt die Person bei Sekundenschlaf. Aus Gründen der besseren Verständlichkeit ist bei manchen Ausführungen von einem Fahrer die Rede. Die Beschreibung und die Art der Anwendung der Erfindung beziehen sich jedoch analog auch auf weitere Anwendungsbereiche, z. B. die Verkehrsraumüberwachung durch Fluglotsen, wobei das System zur Warnung bei Sekundenschlaf des Fluglotsen genutzt werden kann.

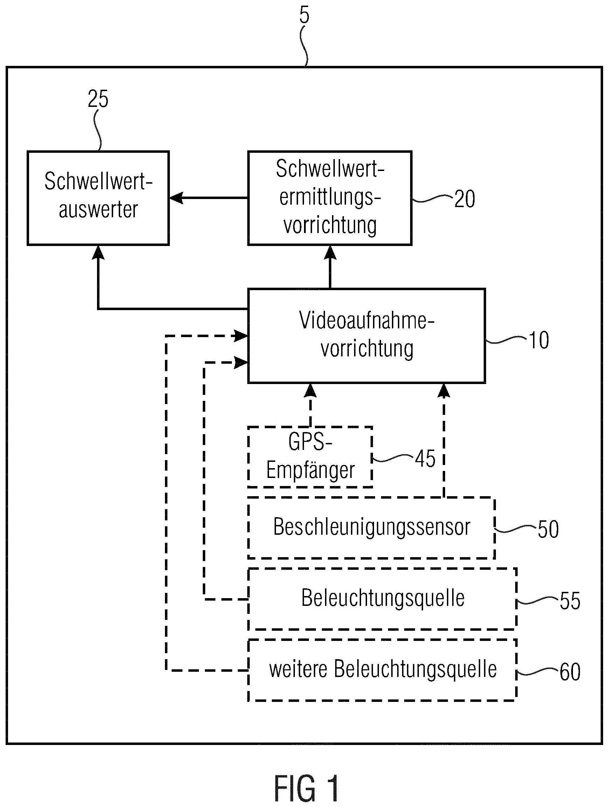

Fig. 1 zeigt ein schematisches Blockdiagramm einer Vorrichtung 5 zur Detektion eines Sekundenschlafs. Die Vorrichtung 5 zur Detektion eines Sekundenschlafs weist eine Videoaufnahmevorrichtung 10, eine Schwellwertermittlungsvorrichtung 20 sowie einen Schwellwertauswerter 25 auf. Die Videoaufnahmevorrichtung 10 zum videobasierten Beobachten einer Person 15 sowie einer Augenpartie der Person 15 ist ausgebildet, eine Bildfolge der Person 15 und der Augenpartie aufzunehmen und an die Schwellwertermittlungsvorrichtung 20 auszugeben. Die Schwellwertermittlungsvorrichtung 20 ist ausgebildet, um einen oder mehrere individuell an die Person angepasste Schwellwerte 80 und 85 aus der Bildfolge abzuleiten. Der Schwellwertauswerter 25 ist ferner ausgebildet, um basierend auf den individuell angepassten Schwellwerten zu entscheiden, ob der Sekundenschlaf der Person eingetreten ist, wobei die individuell angepassten Schwellwerte genutzt werden, um innerhalb des tatsächlichen zeitlichen Verlaufs der Augenöffnung ein Passieren (z. B. Überschreiten bzw. Unterschreiten) der Schwellwerte und damit das Vorliegen des Sekundenschlafes festzustellen. Ferner kann der Schwellwertauswerter 25 zur Feststellung des Sekundenschlafs gemäß voriger Beschreibung ausgebildet sein.

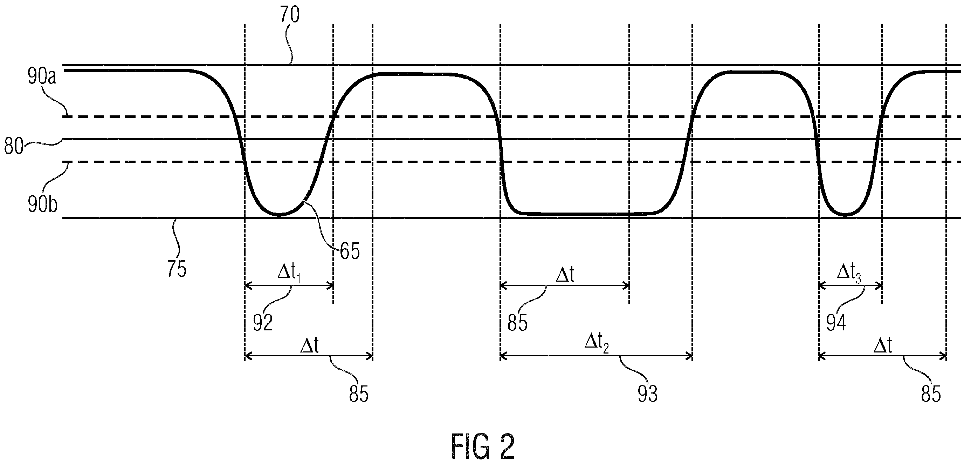

Die Schwellwertermittlungsvorrichtung 20 ist somit ausgebildet, individuell an den Nutzer bzw. die Person 15 und an Umgebungseinflüsse angepasste, zeitlich variable Schwellen zur Detektion von Sekundenschlaf aus der Bildfolge abzuleiten. Umgebungseinflüsse meint insbesondere sich ändernde Lichtverhältnisse wie beispielsweise Sonne, Schatten, bedeckter Himmel, Nacht oder blendende Scheinwerfer eines entgegenkommenden Fahrzeugs. Um die Umgebungseinflüsse in die Bestimmung der individuell angepassten Schwellwerte mit einzubeziehen, ist die Schwellwertermittlungsvorrichtung ausgebildet, basierend auf einer Änderung der Umgebungshelligkeit, die individuell angepassten Schwellwerte an die geänderten Umgebungsverhältnisse anzupassen. Die Schwellen sollen einen für Sekundenschlaf charakteristischen "Augenöffnungsverlauf" beschreiben, indem die eine Schwelle definiert, welche Augenöffnung als geschlossenes Auge angesehen wird und die andere Schwelle definiert, wie lange das Auge als geschlossen erkannt werden muss bis ein Sekundenschlaf festgestellt wird. Die Schwellenwerte werden nachfolgend bezüglich Fig. 2 näher erläutert.

Die individuell angepassten Schwellen 80 und 85 können personenspezifische Merkmale, wie beispielsweise anatomische oder physiologische Merkmale, der Person 15 berücksichtigen. Aus den individuell angepassten Schwellenwerten 80 und 85 kann der Schwellwertauswerter 25 das Eintreten eines Lidschlusses als Indikator für einen eintretenden Sekundenschlaf bestimmen. Dies wird im Folgenden näher erläutert.

Optional kann die Vorrichtung 5 einen GPS-Empfänger 45 und/oder einen Beschleunigungssensor 50 aufweisen. Die Sensoren können eine Fortbewegungsgeschwindigkeit der Vorrichtung 5 erfassen wobei davon ausgehend auf die Geschwindigkeit der Person bzw. des Fahrzeuges geschlossen werden kann. Die Schwellwertermittlungsvorrichtung 20 ist ferner ausgebildet ist, die individuell angepassten Schwellwerte 80 und 85 an die Fortbewegungsgeschwindigkeit anzupassen. So kann beispielsweise bei einem Einsatz in einem Fahrzeug die Geschwindigkeit bzw. der Fahrzustand (fahren/stehen) des Fahrzeugs erkannt und die Sekundenschlafwarnung abgeschaltet werden, da eine Sekundenschlafwarnung bei einem Fahrer vorzugsweise bei einem sich bewegenden Fahrzeug oder allgemein bei einem sich bewegenden Fahrer erforderlich ist.

Weitere Ausführungsbeispiele zeigen die Vorrichtung 5 mit einer Beleuchtungsquelle 55, die Strahlung oberhalb eines für die Person 15 sichtbaren Wellenlängenbereichs aussendet, wobei die Videoaufnahmevorrichtung 10 ausgebildet ist, die Strahlung zu detektieren. Somit kann die Person 15 unabhängig von den äußeren Lichtverhältnissen, beispielsweise auch bei völliger Dunkelheit, beleuchtet werden, ohne dass die Person 15 geblendet oder gestört wird.

Optional kann die Vorrichtung 5 ferner eine weitere Beleuchtungsquelle 60 aufweisen, die von der Beleuchtungsquelle 55 entfernt angeordnet ist. Somit kann die Person 15 von zwei Beleuchtungsquellen 55 und 60 in unterschiedlichen Winkeln beleuchtet werden. Die Videoaufnahmevorrichtung 10 ist ausgebildet, eine Kombination von Bildaufnahmen, bei denen die Person 15 durch aufeinanderfolgendes Ausleuchten der Augenpartie mit der Beleuchtungsquelle 55 und der weiteren Beleuchtungsquelle 60 beleuchtet ist, zu bestimmen, um Reflexe im Bereich der Augenpartie der Person zu vermeiden oder zu vermindern.

Fig. 2 zeigt eine schematische Darstellung eines Verlaufs der Augenöffnung 65 mit individuell ermittelten Referenzwerten 70 und 75 sowie den individuell angepassten Schwellwerten 80 und 85 zur Detektion des Sekundenschlafs. Die Augenöffnung kann dabei eine absolute oder, beispielsweise auf eine Entfernung des Auges zur Videoaufnahmevorrichtung 10, normierte Augenöffnung sein. Der erste individuell angepasste Schwellwert 80 wird zur Ermittlung eines Lidschlusses eines Auges genutzt, wobei der Lidschluss zwischen dem ersten individuell ermittelten Referenzwert 70, der ein geöffnetes Auge beschreibt und dem zweiten individuell ermittelten Referenzwert 75, der ein geschlossenes Auge beschreibt, liegt, und der Schwellwert 80 den Übergang des geöffneten zum geschlossenen Auge (Lidschluss) kennzeichnet. Anders ausgedrückt liegt ein Lidschluss dann vor, wenn ein individuell an den Nutzer angepasster und durch die Umgebungseinflüsse bedingter Grad der Augenöffnung unterschritten ist, der das Auge als geschlossen bewertet.

Der zweite individuell angepasste Schwellwert 85 bezieht sich auf eine zeitliche Dauer des Lidschlusses. Es wird eine individuelle Lidschlusszeit bestimmt, um bei Überschreiten derselben den Lidschluss als Sekundenschlaf zu bewerten. Ein Sekundenschlaf liegt dann vor, wenn beide individuell angepassten Schwellwerte 80 und 85 für die zur Auswertung herangezogenen Augen unterschritten (Augenöffnung) bzw. überschritten (zeitliche Schwelle) wurden. Die Schwellwertermittlungsvorrichtung 20 ist demnach ausgebildet, einen Lidschlag bzw. ein Zwinkern anhand eines Musters im zeitlichen Augenöffnungsverlauf 65 zu ermitteln, wobei das Muster, aus Richtung des ersten individuell ermittelten Referenzwertes 70, ein Unterschreiten des ersten individuell angepassten Schwellwertes 80 und eine Rückkehr in Richtung des ersten Referenzwert 70 innerhalb einer vorgegebenen Zeit Δ*t* aufweist. Je nach Erfassbarkeit der Augen durch die Videoaufnahmevorrichtung 10 werden ein oder beide Augen zur Bestimmung des Sekundenschlafs herangezogen. Dies wird nachfolgend näher erläutert.

Weitere individuell angepasste Schwellwerte können zu weiteren Parametern bzw. Qualitätsmaßen ermittelt werden und als Kriterien für das Vorliegen des Sekundenschlafes in die Auswertung der individuell angepassten Schwellwerte mit einbezogen werden. Die Nutzung solcher Schwellwerte kann z.B. die Erkennung des Sekundenschlafs erleichtern, indem Parameter wie z.B. eine Position, eine Ausrichtung oder eine Größe von markanten Mustern oder Punkten bestimmt wird. Die markanten Muster oder Punkte können z. B. der Kopf, das Gesicht, die Augen, die Iris, die Pupille oder der Pupillenmittelpunkt der Person sein. Die weiteren Beispiele für individuell angepasste Schwellwerte können einzeln oder in beliebiger Kombination, beispielsweise auch mit den vorher genannten individuell angepassten Schwellwerte (Lidschlussgrad und Dauer des Lidschlusses) verwendet werden. Ebenso können sie die vorher genannten individuell angepassten Schwellwerte (Lidschlussgrad und Dauer des Lidschlusses) ersetzen.

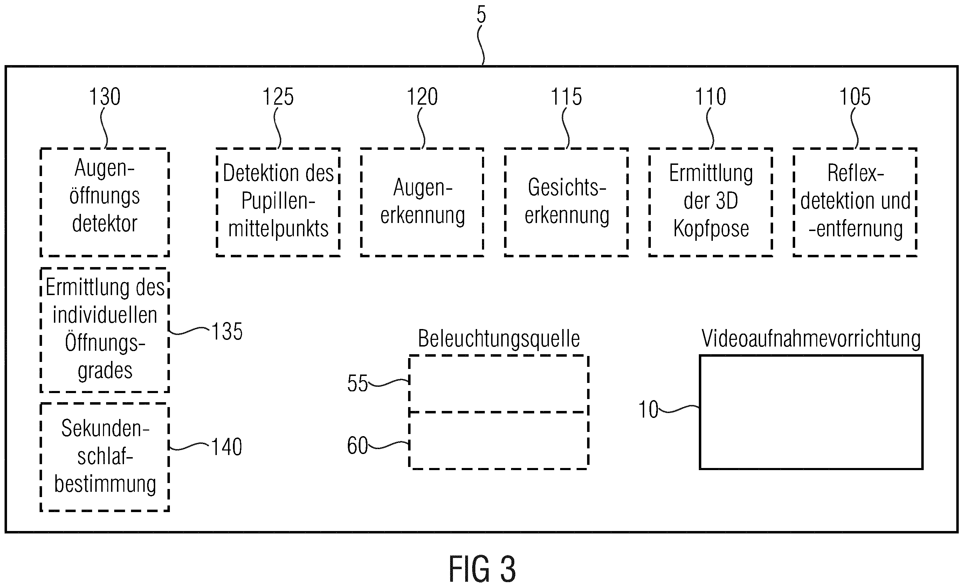

Zur Bestimmung des ersten individuell angepassten Schwellwertes 80 wird die Augenöffnung vermessen und der Lidschluss detektiert. Dies wird für jedes Auge separat durchgeführt. Ein Verfahren zur Vermessung der Augenöffnung wird bezüglich Fig. 3 beschrieben. Das Ergebnis ist ein zeitlicher Verlauf der Augenöffnung 65 (vgl. Fig. 2) in absoluten oder normierten Werten ausgedrückt. Durch die Normierung, beispielsweise als Pixel normiert auf die Distanz des betrachteten Auges der Person 15 zur Kamera 10, kann der Einfluss von unterschiedlichen Distanzen zwischen Fahrer und Gerät eliminiert werden.

Aus dem zeitlichen Verlauf der Augenöffnung 65 inklusive Zwinkern bzw. Lidschläge kann der erste individuell ermittelte Referenzwert 70, auch als oberer Referenzwert bezeichnet, der ein geöffnetes Auge beschreibt und der zweite individuell ermittelte Referenzwert 75, auch als unterer Referenzwert bezeichnet, der ein geschlossenes Auge beschreibt, ermittelt werden. Ferner kann der erste individuell angepasste Schwellwert 80, innerhalb des durch die individuell ermittelten Referenzwerte 70 und 75 beschränkten Bandes, bestimmt werden. Das Augenlid gilt als geschlossen (Lidschluss), wenn die aktuelle Augenöffnung kleiner als der erste individuell angepasste Schwellwert ist. Die Schwellwertermittlungsvorrichtung ist demnach ausgebildet, die individuell angepassten Schwellwerte an die Anatomie und Physiologie des Fahrers anzupassen. Ferner werden die individuell ermittelten Referenzwerte 70 und 75 und die individuell ermittelten Schwellwerte 80 und 85 kontinuierlich durch die Auswertung auftretender Lidschläge während des Betriebs durch die Schwellwertermittlungsvorrichtung 20 angepasst.

Weiterhin kann der erste individuell angepasste Schwellwert 80 eine Hysterese 90a, 90b, aufweisen, die ausgebildet ist, einen Lidschluss bei einem geringeren Augenöffnungswinkel 90b beim Übergang vom geöffneten zum geschlossenen Auge und bei einem größeren Augenöffnungswinkel 90a beim Übergang vom geschlossenen zum geöffneten Auge festzulegen.

Die individuell ermittelten Referenzwerte 70 und 75 für ein offenes bzw. geschlossenes Auge, individuell bezogen auf die Person 15 und die Umgebungsbedingungen, werden durch die Auswertung der, beispielsweise spontanen bzw. unwillkürlichen, Lidschläge der beobachteten Person fortlaufend ermittelt. Dies ist vorteilhaft, da dadurch individuelle anatomische und physiologische Merkmale der Person 15 berücksichtigt werden. Darüber hinaus ist das Verfahren resistent gegen Störungen durch Umgebungseinflüsse auf die beobachtete Person 15, wie beispielsweise grelles Licht, da die Schwell- bzw. Referenzwerte dynamisch angepasst werden und sich damit an geänderte Situationen adaptieren. Die Auswertung kann auf Basis einer Erkennung von spontanen Lidschlägen bzw. spontanem Zwinkern (engl. blinks), deren "Auf"- und "Zu"-Zustände als individuell offenes bzw. geschlossenes Auge betrachtet werden, erfolgen.

Ein Verfahren zur Erkennung von Zwinkern bzw. Lidschlägen wird nachfolgend beispielhaft erläutert. Weiterhin sind jedoch auch andere Verfahren einsetzbar. Nachdem ein Lidschlag abgeschlossen ist wird derselbe als ein in gewissen Grenzen variables Muster anhand der Signalform im zeitlichen Verlauf der Augenöffnung 65 erkannt. Das Muster umfasst eine Verringerung der Augenöffnung und eine anschließende Vergrößerung der Augenöffnung etwa auf den Anfangswert 70, beides in kurzer Aufeinanderfolge. Die Maxima und Minima dieser Signalverläufe, die zur besseren Auswertbarkeit gefiltert sein können, werden als oberer und unterer Referenzwert 70 und 75 genutzt.

Gemäß einem weiteren Ausführungsbeispiel kann die Schwellwertermittlungsvorrichtung den individuellen Referenzwert jeweils für ein individuell "geöffnetes" und ein individuell "geschlossenes" Auge ermitteln. Diese beiden individuellen Referenzwerte beschreiben das Auge als individuell "offen" oder "geschlossen". Zur Bestimmung wird ein individueller Trend dieser beiden Referenzwerte aufgezeichnet, d. h. es wird fortlaufend erfasst, wie viel das individuelle "geöffnet" (entspricht dem ersten individuell angepassten Referenzwert 70) und individuelle "geschlossen" (entspricht dem zweiten individuell ermittelten Referenzwert 75) der Person 15 ist, und der erste individuelle Schwellwert 80 zwischen diesen beiden individuellen Referenzwerten festgelegt (Lidschluss). Anders ausgedrückt definiert sich ein geschlossenes Lid prozentual am individuellen "offen" bzw. "geschlossen".

Aus den individuellen Schwellwerten lässt sich ein individueller Sekundenschlaf bestimmen. Die im Vorhinein bestimmte adaptive Schwelle 80, ab der der Augenöffnungsgrad als kritisch bewertet wird, wird um eine Hysterese 90a, 90b ergänzt. Die Schwelle ist adaptiv, da sie auf den individuellen Referenzwert für das individuell "geöffnete" und "geschlossene" Auge bezogen ist. Das Prinzip wird bezüglich Fig. 3 näher erläutert.

Zur Feinjustierung des ersten individuell angepassten Schwellwertes 80 kann auch die Lidschlussgeschwindigkeit berücksichtigt werden. Diese kann beispielsweise mittels der Dauer vom Verlassen des ersten individuell ermittelten Referenzwertes 70 bis zum Erreichen es zweiten individuell ermittelten Referenzwertes 75 ermittelt werden. Wird der individuell angepasste Schwellwert 80 z. B. mittig zwischen dem oberen und dem unteren Referenzwert 70 und 75 angeordnet, so kann die Lidschlussgeschwindigkeit dahingehend genutzt werden, dass der individuell angepasste Schwellwert 80 weiter nach oben (in Richtung Referenzwert 70) oder nach unten (in Richtung Referenzwert 75) korrigiert wird.

Gemäß einem weiteren Ausführungsbeispiel werden auch die Referenzwerte 70 und 75 feinjustiert. Dies erfolgt unter Hinzuziehung der Kopfpose, das ist die Position bzw. räumliche Lage und die Ausrichtung des Kopfes, welche mittels sogenannten Head-Pose Trackings (dt.: Kopfposen Verfolgung) ermittelt werden kann. Die Schwellwertermittlungsvorrichtung 20 ist demnach ausgebildet, den individuell angepassten Schwellwert 80 anhand der ermittelten Kopfpose anzupassen und, abhängig von der Kopfpose, den Lidschluss anhand eines Auges oder anhand beider Augen der Person 15 zu bestimmen. Insbesondere der erste individuell ermittelte Referenzwert kann durch eine veränderte Perspektive, unter der das Auge aufgenommen wurde, ungenau bestimmt sein, wenn beispielsweise der Augenmittelpunkt, bei dem die Augenöffnung durch die Krümmung des Augenlides am größten ist, für die Videoaufnahmevorrichtung 10 verdeckt ist. Durch das Head-Pose Tracking kann demnach bestimmt werden, ob die Person 15 frontal auf die Videoaufnahmevorrichtung 10 schaut oder alternativ eine Korrektur der Referenzwerte 70 und 75 vorgenommen werden muss. Die Kopfpose kann demnach unterstützend zur Sekundenschlaferkennung herangezogen werden und somit optional zur Verbesserung der Augenauswertung genutzt werden.

Basierend auf der Auswertung der aufgenommenen Videodaten kann ein 3D-Kopfmodell an die Form, Lage und Ausrichtung des beobachteten Kopfes angepasst werden und basierend auf der Kenntnis der Form, Lage und Ausrichtung können die Schwellwerte individuell angepasst werden. Die Vorrichtung 5 kann demnach einen 3D-Modellierer umfassen, der basierend auf einer Auswertung der individuell angepassten Schwellwerte ein dreidimensionales Modell eines Kopfes der Person berechnet. Zur Vereinfachung kann der 3D-Modellierer auch mit einer vereinfachten Repräsentation der Form, Lage und Ausrichtung des dreidimensionalen Modells des Kopfes, z. B. auf Basis eines Zylinders, rechnen. Das Head-Pose Tracking wird bezüglich Fig. 3 detaillierter beschrieben.

Der zweite individuell angepasste Schwellwert 85, also eine Zeitdauer Δ*t* des Lidschlusses, wird für beide Augen gemeinsam bestimmt und herangezogen, sofern sich beide Augen im Bild befinden und ausgewertet werden können. Der Schwellwert 85 wird dynamisch, ausgehend von einem individuell ermittelten zeitlichen Maximum der Lidschlussdauer bestimmt, wobei die Lidschlussdauer beispielsweise durch ein Vielfaches der Zeit eines spontan auftretenden Lidschlages bzw. Zwinkerns (gekennzeichnet durch Lidschluss und gleich darauf Lidöffnung) oder auf Basis eines Literaturwertes, bestimmt werden kann. Der Literaturwert kann als Anhaltspunkt, um den Wert nicht unrealistisch hoch zu definieren, bzw. als Initialisierungswert dienen, für den Fall, dass die individuelle Lidschlusszeit noch nicht ermittelt ist. Optional kann der zweite individuell angepasste Schwellwert 85 auch von der Fortbewegungsgeschwindigkeit der Person, beispielsweise in einem Auto, abhängen. So ist eine längere Lidschlusszeit bei einer hohen Geschwindigkeit gefährlicher als bei einer niedrigen Geschwindigkeit, da in der gleichen Zeit eine größere Strecke mit geschlossenen Augen zurückgelegt wird. Hierzu können die Daten des GPS-Empfängers 45 bzw. des Beschleunigungssensors 50 herangezogen werden. Ferner kann das zeitliche Minimum des zweiten individuell angepassten Schwellwertes 85 an die Zeitdauer des auftretenden Lidschlags bzw. Zwinkerns angepasst werden.

Der zweite individuell angepasste Schwellwert 85 wird innerhalb des Bandes zwischen dem im Vorhinein beschriebenen Maximum und Minimum angegeben und kann je nach eingestellter Sensitivität hinsichtlich der Sekundenschlafwarnung eingestellt werden. Bei einer hohen Sensitivität (bei gleichzeitiger geringer Spezifität) liegt die Schwelle nahe am zeitlichen Minimum und bei einer geringen Sensitivität (bei gleichzeitig höherer Spezifität) liegt die Schwelle nahe am zeitlichen Maximum. Die Einstellung kann je nach Bedarf im Bereich von "hohe Sensitivität" bis "geringe Sensitivität" gewählt werden. Der Bedarf einer hohen Sensitivität liegt beispielsweise bei einer hohen Fortbewegungsgeschwindigkeit der Person vor.

In Fig. 2 ist beispielhaft der Augenöffnungsverlauf 65 in den Grenzen der individuell ermittelten Referenzwerte 70 und 75 gezeigt. Der adaptive Schwellwert 80 ist mittig zwischen den individuellen Referenzwerten festgelegt und bestimmt den Lidschluss der Person 15, wobei vereinfacht dargestellt ein Lidschluss vorliegt, wenn der Augenöffnungsverlauf den Schwellwert 80 unterschreitet. Um den Schwellwert 80 ist der Hysteresebereich zwischen den Grenzen 90a und 90b definiert, der den Start- sowie Endzeitpunkt des Lidschlusses und somit die Lidschlussdauer bestimmt. Bei unterschreiten der Grenze 90b wird das Auge als geschlossen und bei überschreiben der Grenze 90a als geöffnet bewertet. Liegt die Lidschlussdauer über dem festgelegten Schwellwert 85 (Δ*t*) ist der Sekundenschlaf eingetreten, andernfalls liegt ein Zwinkern bzw. ein spontan auftretender Lidschluss vor.

In anderen Worten definieren die individuell angepassten Schwellwerte 80 und 85 ein Muster, das zur Detektion des Sekundenschlafs mit dem tatsächlichen Augenöffnungsverlauf verglichen wird, um bei Übereinstimmung zwischen dem Muster und dem tatsächlichen Augenöffnungsverlauf (entspricht einem Überschreiten bzw. Unterschreiten der Schwellwerte) den Sekundenschlaf zu detektieren. Bezogen auf Fig. 2 wird bei Unterschreiten der zweiten Hysteresegrenze 90b ein Zeitnehmer zur Bestimmung des zweiten angepassten Schwellwertes 85 gestartet. Dieser läuft so lange, bis die erste Hysteresegrenze 90a wieder überschritten wird. Ist die Dauer zwischen (zuerst) Unterschreitung und (anschließend) Überschreitung der Hystereseschwellen kürzer als der zweite Schwellwert 85 (Δ*t*) liegt ein spontan auftretender Lidschluss bzw. ein Zwinkern vor, wie es Anhand der beispielhaften Zeitdauern Δ*t*₁ 92 und Δ*t*₃ 94 gezeigt ist. Ist die Dauer der Unter- bzw. Überschreitung größer oder gleich dem zweiten individuell angepassten Schwellwert 85, liegt ein Sekundenschlaf vor. Dies ist Anhand des beispielhaften Lidschlusses mit der Dauer Δ*t*₂ 93 gezeigt.

Anders ausgedrückt tritt ein Sekundenschlaf dann ein, wenn (vereinfacht ausgedrückt) beide Lider zu einem definierten Grad x für eine definierte Zeit Δ*t* geschlossen sind. Je nach Kopflage, z. B. wenn nur eines der beiden Augen von der Videoaufnahmevorrichtung 10 erfasst wird, ist auch die Auswertung basierend auf diesem einen Auge möglich. Ferner sollte eine Detektion eines Sekundschlafs sofort nach dem starten der Auswertung verfügbar sein. Hierfür kann z. B. auch eine höhere Unsicherheit bzw. eine hohe Sensitivität in Kauf genommen werden, da am Anfang einer Messung wenige oder noch keine individuellen Daten über die beobachtete Person vorliegen.

Für die Ableitung der beschriebenen Detektionsschwellen 80 und 85 und der Referenzwerte 70 und 75 werden individuelle anatomische und physiologische Merkmale (bspw. Individuelle anatomische Augenöffnung) und individuelle Parameter wie Lidschluss, Kopfneigung usw. herangezogen.

Ferner kann die 3D-Head-Pose genutzt werden, um festzulegen, welches der beiden Augen oder ob ggf. beide Augen zur Auswertung genutzt werden. Je nach Lage und Verdrehung des Kopfes in Bezug zur Kamera wird entschieden, welches der beiden Augen und ob ggf. beide Augen zu Sekundenschlafdetektion herangezogen werden. Ermittelt das Head-Pose Tracking eine ausreichend frontale Ausrichtung des Kopfes in Bezug zur Kamera werden beide Augen zur Auswertung herangezogen.

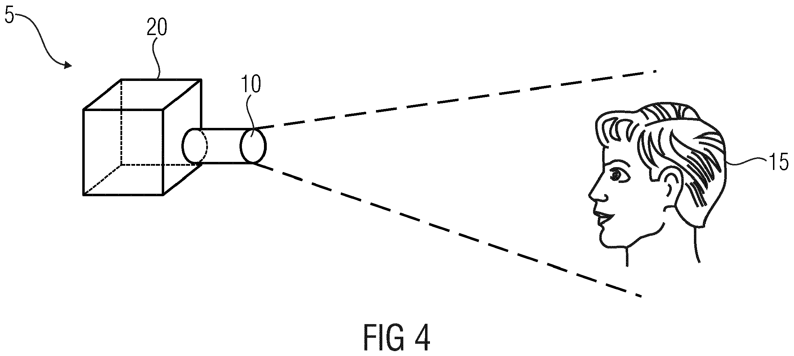

Fig. 3 zeigt ein schematisches Blockdiagramm einer Vorrichtung 5 zur Detektion eines Sekundenschlafs in einer von Fig. 1 abweichenden Darstellung, die detailliertere Funktionsblöcke aufweist. Die Vorrichtung 5 zur Detektion eines Sekundenschlafs umfasst eine Videoaufnahmevorrichtung 10 zum videobasierten Beobachten einer Person 15 sowie einer Augenpartie der Person 15. Ferner umfasst die Vorrichtung 5 zur Detektion eines Sekundenschlafs optional eine Beleuchtungsquelle 55 sowie optional weitere Funktionsblöcke 105 - 140. Die Funktionsblöcke können je nach Implementierung der Schwellwertermittlungsvorrichtung 20, dem Schwellwertauswerter 25 oder einer separaten Rechenvorrichtung (nicht gezeigt) zugeordnet werden. Gemäß Ausführungsbeispielen ist die Vorrichtung 5 in Bezug auf die Person 15 kontaktfrei (siehe Fig. 4). Kontaktfrei bedeutet, dass, bei bestimmungsgemäßem Gebrauch, kein Teil der Vorrichtung 5 mit der Person 15 in Berührung ist.

Die Videoaufnahmevorrichtung 10 ist ausgebildet, eine Bildfolge der Person 15 und der Augenpartie aufzunehmen und an die Funktionsblöcke auszugeben um zu entscheiden, ob der Sekundenschlaf der Person 15 eingetreten ist.

Die Videoaufnahmevorrichtung 10 ist ferner ausgebildet, um die Person 15 oder zumindest ein Gesicht der Person 15 aufzunehmen und eine Bildfolge der Aufnahme an die Funktionsblöcke 105 - 140 auszugeben. Die Funktionsblöcke führen, basierend auf der Bildfolge, Bilderkennungs- und Bildverarbeitungsschritte durch.

Ausführungsbeispiele zeigen die Entfernung von Reflexen 105 in der Bildfolge. Reflexe können beispielsweise an Brillen (-gläsern) oder den Pupillen bzw. den Augen (insbesondere auf der Cornea) auftreten. Reflexe können algorithmisch entfernt werden, indem der Reflex detektiert, segmentiert und mit interpoliertem oder wenn möglich rekonstruiertem Bildinhalt aufgefüllt wird. Ferner können Reflexe vermieden werden, indem die Person mittels Licht, beispielsweise aus dem Infrarotspektrum, abwechselnd von zwei verschiedenen Positionen beleuchtet wird. Zwei aufeinanderfolgende Einzelbilder können so miteinander verrechnet werden, dass die Reflexe deutlich reduziert sind, da die auftretenden Reflexe bzw. Spiegelungen durch die unterschiedliche Beleuchtung an unterschiedlichen Bildpositionen auftreten. Ein weiteres Ausführungsbeispiel beschreibt eine Modifikation des letztgenannten Algorithmus. Es werden wiederum zwei Lichtquellen, z. B. infrarot LEDs (IR-LEDs), abwechselnd zur Ausleuchtung eingesetzt und fortlaufend Reflexe detektiert, wobei die Reflexe nicht ausgebessert bzw. interpoliert werden müssen. Liegen die Reflexionen in einer Region of Interest (ROI, dt.: Bereich von Interesse), beispielsweise über dem Auge, so kann die andere vorhandene Lichtquelle bzw. IR-LED verwendet werden. Auf diese Weise kann fortlaufend zwischen den Lichtquellen hin- und hergewechselt und die jeweils vorteilhafteste Beleuchtung gewählt werden.

Ausführungsbeispiele zeigen die Gesichtserkennung 115. Die Gesichtserkennung 115 kann Gesichter oder Bestandteile eines Gesichtes, wie beispielsweise Augen, Nase, Mund, Ohren und/oder allgemeiner Punkte (bzw. Landmarks) im Gesicht oder auf der Gesichtskontur, beispielsweise mit einem Kaskadendetektor auffinden, um eine zweidimensionale (2D) Position und Größe des Gesichts sowie eine Position der Augen (oder andere Gesichtsbestandteile) zu bestimmen. Ein Kaskadendetektor, z. B. nach Viola/Jones [2], kann verschiedene Signalverarbeitungsalgorithmen und/oder gleiche Signalverarbeitungsalgorithmen mit unterschiedlichem Detaillierungsgrad bzw. Trainingsgrad umfassen, um die allgemeinen Merkmale auszuwerten. Die Verwendung von Haar-like Features (dt.: Haar-ähnliche Merkmale), beispielsweise in Viola Jones [2] gezeigt, ist für das beschriebene Ausführungsbeispiel optional. Zunächst kann so mittels einfacher Algorithmen eine Vorklassifikation durchgeführt werden, sodass spezifischere Algorithmen nur noch auf einem verkleinerten Datensatz angewendet werden brauchen und dadurch die Rechenzeit reduziert wird. Ferner können anstelle des Kaskadendetektors auch andere Signalverarbeitungsverfahren wie z. B. neuronale Netze eingesetzt werden.

Weitere Ausführungsbeispiele zeigen die Durchführung einer Augenerkennung 120 des Fahrers bzw. der Person 15. Die oben beschriebene Gesichtserkennung kann in diesem Schritt verfeinert werden, indem eine analysierte 2D-Position, z. B. der Augenmittelpunkt der Augen, bei geöffneten oder geschlossenen Augen bestimmt wird. Weiterhin kann eine Schätzung zur Öffnung der detektierten Augen vorgenommen werden. Die Augenerkennung kann wiederum mit einem Kaskadendetektor ausgeführt werden.

Ein weiteres Ausführungsbeispiel beschreibt die Detektion der Pupille bzw. des Pupillenmittelpunktes 125. Der Pupillenmittelpunkt kann insbesondere bei geöffneten Augen bestimmt werden und verfeinert dann die Position aus der Augenerkennung 120. Die Pupille bzw. der Pupillenmittelpunkt kann beispielsweise mittels eines gradientenbasierten Verfahrens [1] ermittelt werden. Die dunkle Pupille und die ebenfalls dunkle Regenbogenhaut bzw. Iris heben sich deutlich von dem weißen Augapfel ab und bilden somit einen starken Gradienten, der z. B. mit dem in [1] beschriebenen Verfahren detektiert werden kann.

Ergänzend oder alternativ zeigen Ausführungsbeispiele die Vorrichtung 5 mit einem 3D-Kopf-Modellierer zur Ermittlung der 3D Kopfpose 110, der ein dreidimensionales Modell eines Kopfes der Person bzw. eine 3D Kopfpose berechnet. Durch die Hinzunahme der 3D Kopfpose kann eine robustere und genauere Detektion des Kopfes bzw. der Kopflage und Kopfposition erreicht werden. Zudem können durch ein 3D Modell Landmarks (dt.: Orientierungspunkte) in der Augen-ROI zur Ermittlung des Augenöffnungsgrades ermittelt werden. Weiterhin kann die Kopfneigung bei der Lidauswertung ebenso berücksichtigt werden, wie die Kopfdrehung, um festzustellen, welches Auge zuverlässiger ausgewertet werden kann. Die Kopfneigung kann ferner herangezogen werden, um die individuellen Referenzwerte 70 und 75 und die adaptiven Schwellwert 80 anzupassen. Somit ist die Vorrichtung 5 ausgebildet, eine Position des Auges der Person 15 zu bestimmen.

Zur Bestimmung der 3D Kopfpose kann zunächst eine Initialisierung erfolgen. Diese umfasst eine grobe 2D Gesichtsdetektion mittels eines Kaskadendetektors (s. Gesichtsdetektion 115). Weitere Gesichtsbestandteile können ebenfalls über einen Kaskadendetektor, wie bereits für die Gesichts- und Augenerkennung beschrieben, aufgefunden werden. Dies können die Augenposition, die Nasenspitze oder die Mundwinkel sein. Ausgehend von den grob klassifizierten Gesichtsbestandteilen kann ein 2D Gitternetzmodell über das Gesicht gelegt bzw. aufgezogen und mit Hilfe von z. B. eines ASM (Active Shape Modell, dt.: adaptives Konturmodell bzw. Formmodell) [3] bzw. AAM (Active Appearance Model, dt.: adaptives Erscheinungsmodell) [4] basierten Verfahrens an die Person 15 angepasst werden. Hieraus können Landmarks (dt.: Orientierungspunkte) gewonnen werden, die für die weitere Verarbeitung nützlich sind. Die Landmarks können mittels Projektion auf einen Normkopf bzw. auf Basis eines Normkopfes in den 3D-Raum zurückgerechnet werden. Dies kann z. B. mittels POSIT (POS with ITerations, dt.: Positionsermittlung mittels Iterationen) [5] erfolgen.

Nach der Initialisierung können innerhalb des modellierten Gesichts 2D Referenzpunkte (Featurepunkte) gesucht und verfolgt werden (Feature Tracking). Diese können mit den oben beschriebenen Landmarks übereinstimmen, müssen es aber nicht. Alternativ oder ergänzend werden jedoch auch abweichende Referenzpunkte ermittelt, da die Referenzpunkte häufig nicht mit den Landmarks übereinstimmen. Auf Basis der Initiallage des Kopfes sowie der Verfolgung der Referenzpunkte kann eine Positionsverfolgung des Kopfes erfolgen. Zur Vereinfachung, z. B. zur Reduzierung des Rechenaufwands, kann die Oberfläche des Kopfes auf einen 3D Zylinder heruntergebrochen werden, der mit der zuvor ermittelten Initiallage des Kopfes initialisiert ist. Anders ausgedrückt kann der 3D-Kopf-Modellierer 110 eine Abschätzung der dreidimensionalen Lage und Ausrichtung des Kopfes, also einer 3D-Kopfpose, auf Basis eines Zylinders berechnen.

Zur weiteren Verfolgung werden 2D Featurepunkte entsprechenden 3D Positionen auf dem Zylinder zugeordnet. Hieraus resultiert eine räumliche Lage und Ausrichtung in sechs Freiheitsgraden (6 DOF, engl.: Degrees of Freedom) der Punktwolke. Die 2D Featurepunkte werden über die Zeit hinweg, also von Bild zu Bild, verfolgt (engl.: getrackt) und deren räumliche Lage ermittelt. Fallen Featurepunkte weg bzw. kommen neue Featurepunkte hinzu, beispielsweise durch eine Drehung des Kopfes, wird deren räumliche Position auf dem 3D Zylindermodell ermittelt. Gehen zu viele 2D Featurepunkte verloren, kann die Lage des 3D Zylinders nicht mehr nachgeführt werden und es ist eine erneute Initialisierung erforderlich. Wie oft eine erneute Initialisierung erforderlich wird hängt von der Robustheit der 2D Featurepunkte und der Verfolgung der Featurepunkte bzw. des Featuretrackings ab. Die Robustheit bzw. Störanfälligkeit der Featurepunkte ist z. B. abhängig von Beleuchtungsänderungen, perspektivischen Verzerrungen oder kurzen Verdeckungen. Um zeitlichen Drifts (dt.: langsames Verschieben) entgegen zu wirken, kann zwischendurch ein Abgleich mit den Landmarks bzw. der 3D Position aus der Initialisierung gemacht werden, d. h. die Initialisierung wird von Zeit zu Zeit parallel zum Tracking durchlaufen. Ferner können die Landmarks aus der Initialisierung in 3D bei der Positionsverfolgung des Kopfes mitgeführt und deren 2D Bestimmung ermittelt und zum Abgleich genutzt werden.

Weitere Ausführungsbeispiele zeigen die Vorrichtung 5 mit einem Augenöffnungsdetektor 130, der eine Ermittlung bzw. einen zeitlichen Verlauf der Augenöffnung, ausgedrückt in normierten Werten z. B. Pixel normiert auf die Distanz zur Kamera, bestimmt. In anderen Worten bestimmt der Augenöffnungsdetektor 130 fortlaufend eine aktuelle Weite der Augenöffnung. Zur Augenanalyse werden ein oder mehrere Landmarks innerhalb der Augen-ROI genutzt. Für den Fall eines einzelnen Landmarks kann die Position aus der anfänglichen Augenerkennung (s. Funktionsblöcke Gesichtserkennung 115 bzw. Augenerkennung 120) genutzt werden, die beispielsweise mit dem Kaskadendetektor ermittelt wurde. Steht nur ein einzelner Landmark zur Verfügung, ist es vorteilhaft, wenn dies der Augenmittelpunkt ist. Weitere wichtige Landmarks stellen z. B. die Augenecken und das obere sowie untere Augenlid dar. Diese Landmarks sind jedoch optional, und können unterstützend zur Augenanalyse durch den Augenöffnungsdetektor herangezogen werden. Das hat den Vorteil, dass die Augenanalyse auch durchgeführt werden kann, wenn die 3D Kopfverfolgung (3D Head Pose Tracking) einmal versagt und keine Landmarks geliefert werden können und somit nur eine geschätzte Augenposition z. B. der Augenmittelpunkt aus dem 2D-Tracking von Funktionsblock 115, 120 oder 125 vorliegt. Demnach ist der Augenöffnungsdetektor 130 ausgebildet, basierend auf einem oder mehreren Landmarks aus den Funktionsblöcken 110 bis 125, eine Augenöffnung zu bestimmen. Die Ermittlung der Augenöffnung erfolgt mit Hilfe eines Templates (dt.: Modell, Schablone). Der Augenöffnungsdetektor 130 kann die Augenöffnung mittels eines Templates modellieren, das z. B. über vier Punkte bzw. Landmarks beschrieben ist und darüber an das detektierte Auge angepasst wird. Der Randbereich zwischen den vier Landmarks wird mittels mathematischen Kurven interpoliert. Der Augenöffnungsdetektor ist somit ausgebildet, den Augenöffnungswinkel mittels einer Kurvenapproximation zu bestimmen, die zwischen zumindest vier den Augenumriss beschreibende Parametern interpoliert. Ferner kann das Template vor dem Aufspannen perspektivisch verzerrt werden, sofern die Informationen über 3D Lage und Ausrichtung des Kopfes aus dem Funktionsblock 110 vorliegen.

Im Folgenden werden zwei Verfahren zur Ermittlung des Lidschlussgrades bzw. der Augenöffnung beschrieben, bei denen das Template auf den darunterliegenden Bildinhalt abgestimmt wird. Die Abstimmung bzw. Feinjustage erfolgt über das Gradientenbild und andere geeignete Merkmale, z. B. Eckenmerkmale oder Bogenmerkmale, wie bereits im Vorhinein beschrieben.
1. Abarbeitung mit Augenmittelpunkt als einzigem Landmark: Wenn der Augenmittelpunkt der einzige Landmark ist, kann ein oberer und ein unterer Punkt der Augenöffnung z. B. mit Hilfe einer horizontalen Projektionsfunktion (in Anlehnung an z. B. [6]) geschätzt werden. Das zuvor beschriebene Template wird über den Augenmittelpunkt und den oberen und unteren Punkt der Augenöffnung aufgespannt, wobei sich die Breite des Templates nach der Breite des Gesichts richtet.
2. Abarbeitung mit mehreren Landmarks in der Augen-ROI: Das Template wird entsprechend den Landmarks innerhalb des Bereichs der Augen, d. h. der Augen-ROI, aufgespannt. Danach erfolgt die Ausrichtung und Anpassung des Templates an die Augenöffnung. Zur Ausrichtung und Anpassung des Templates kann z. B. die Ähnlichkeit zum Gradientenbild herangezogen werden.

Ferner kann die Fahrzeugbewegung z. B. im Automobilbereich über einen Beschleunigungssensor integral gemessen werden. Beschleunigungen treten beim Fahren durch Beschleunigen, Bremsen, unterschiedliche Straßenneigung und in Kurven auf. Ein Vibrieren des Autos im Leerlauf bzw. im Stand kann herausgefiltert werden, beispielsweis durch Filtern einer harmonischen Schwingung. Ergänzend oder alternativ kann zur Erfassung der Fahrzeugbewegung GPS (Global Positioning System, dt.: Globales Positionsbestimmungssystem) eingesetzt werden. Dies ist vorteilhaft, wenn die Sekundenschlafwarnung nur durchgeführt werden soll, wenn das Fahrzeug auch in Bewegung ist (Fehlalarme bei stehendem Fahrzeug werden so vermieden). Außerdem kann die Geschwindigkeit des Fahrzeuges herangezogen werden, um die zeitliche adaptive Schwelle 85 zur Sekundenschlafwarnung fein zu justieren.

Die beschriebene Vorrichtung 5 betrifft ein kamerabasiertes und kontaktfreies (bzgl. des Nutzers) System, dessen Kamera bzw. Videoaufnahmevorrichtung 10 auf eine Person 15 ausgerichtet wird. Fig. 4 zeigt dieses Szenario mit einer auf die Person 15 ausgerichteten Videoaufnahmevorrichtung 10. Die Person kann z. B. der Führer eines Fahrzeuges sein. Ferner ist die Vorrichtung 5 dazu geeignet, die individuell angepassten Schwellwerte 80 und 85 sowie die individuell ermittelten Referenzwerte 70 und 75 zur Detektion des Sekundenschlafs der Person in einem Kamera-Livebild zu bestimmen und die Person 15 bei Sekundenschlaf zu warnen. Der Begriff Sekundenschlafwarner ist mehr als Bezeichnung der Erfindung statt als exakte Abgrenzung zu sehen, da damit Mikroschlaf, Sekundenschlaf und auch das Auftreten von länger anhaltendem Schlaf erkannt wird.

Das Verfahren und die genutzten Algorithmen sind dadurch gekennzeichnet, dass der Sekundenschlaf der Person mit Hilfe direkter videobasierter Beobachtung und einer sich individuell an die Person und die Lichtverhältnisse adaptierenden Auswertung erkannt wird. Im Vergleich zu den bestehenden Verfahren, die Sekundärdaten hinsichtlich des Schlafzustandes der Person, wie z. B. auf dem CAN-Bus im Fahrzeug vorhandene Informationen über das Lenk-, Brems- und Beschleunigungsverhalten, bestimmen, werden Primärdaten (die Augenöffnung) ausgewertet. Dadurch können das System bzw. die Algorithmen deutlich präziser und zuverlässiger den Sekundenschlaf erkennen und bei dessen Auftreten warnen, wodurch insbesondere in sicherheitskritischen Bereichen (z. B. Führen eines Fahrzeuges, Verkehrsraumüberwachung (z. B. durch Fluglotsen), Kraftwerksbedienung und -überwachung) Unfälle vermieden und Menschenleben gerettet werden können.

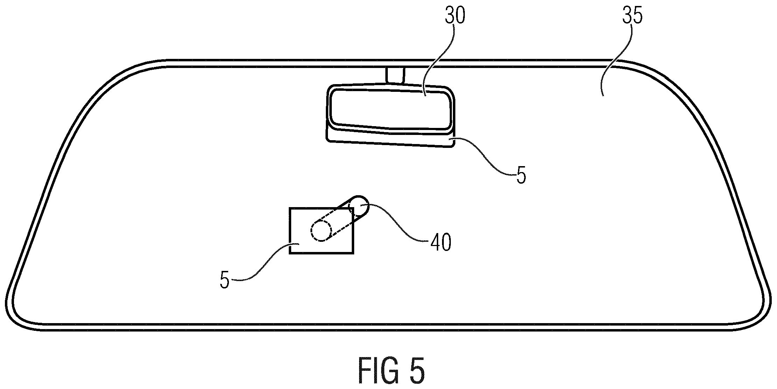

Bezogen auf den Automobilbereich kann die Vorrichtung 5 beispielsweise in den Rückspiegel 30 in einem Auto integriert oder alternativ z. B. als Nachrüstlösung (wie ein Nachrüstnavigationsgerät) mittels einer Halterung am Armaturenbrett, an einem Frischluftgrill oder an der Windschutzscheibe befestigt sein. In Fig. 5 sind zwei dieser Anordnungen gezeigt, wobei die Vorrichtung 5 in den Rückspiegel 30 integriert und damit ebenso an einer Windschutzscheibe 35 angebracht ist, wie die ebenfalls gezeigte Vorrichtung 5 die als Nachrüstlösung mittels der Halterung 40 (z. B. einer Saugnapfhalterung) an der Windschutzscheibe 35 befestigt ist. Ebenso ist eine Integration in das Armaturenbrett, die A-Säule oder unter dem Dach des Autos möglich. Wie bereits beschrieben ist die Vorrichtung jedoch nicht auf den Automobilbereich beschränkt, sondern kann auch in anderen Bereichen eingesetzt werden. Ferner ist eine Position, aus der die Vorrichtung leicht schräg von unten auf das Gesicht schaut, vorteilhaft, da die Vorrichtung in dieser Position meist freie Sicht auf die Augen hat und nur selten von beispielsweise den Augenhöhlen oder Augenbrauen verdeckt sind.

Die Vorrichtung 5 kann als autonomes Gerät, gemäß einem Ausführungsbeispiel auch tragbar oder als Teil eines übergeordneten Assistenz- oder Überwachungssystems ausgeführt sein. Als einfache Variante kann die Vorrichtung als Plug-and-Play Lösung verwendet werden, d. h. der Einbau kann von jedem durchgeführt werden. Dabei ist nur zu beachten, dass die Videoaufnahmevorrichtung auf den Fahrer ausgerichtet ist. Neben der Erfassung der zu beobachtenden Person 15, z. B. ein PKW-Fahrer, ein LKW-Fahrer oder ein Fluglotse, mit einer Kamera, d. h. der Videoaufnahmevorrichtung 10, ist auch ein System mit mehreren Kameras realisierbar. Dies kann die Robustheit erhöhen, indem der Erfassungsbereich (bei einer Kamera kann ein Auge verdeckt sein, dass dann durch eine an einer anderen Stelle positionierte zweite Kamera erfasst wird) erweitert wird oder zusätzliche Parameter (z. B. die Blickrichtung der Person 15) mit in die Auswertung einbezogen werden.

Um unabhängig von äußeren Beleuchtungsverhältnissen zu sein, kann eine Beleuchtung im für den Menschen nicht sichtbaren und damit nicht störenden Spektralbereich, z. B. im nahen Infrarotbereich eingesetzt werden. Ferner kann eine Anzeige des Systemzustanden beispielsweise mittels unterschiedlich farbigen LEDs und die Warnung bei Sekundenschlaf über ein akustisches Signal erfolgen.

Ferner weist die beschriebene Vorrichtung 5 folgende Vorteile auf:
- Lidschlussbestimmung durch direkte, kontaktfreie, videobasierte Messung und daraus die Ableitung der individuell angepassten Schwellwerte 80 und 85 sowie der individuell ermittelten Referenzwerte 70 und 75 zur Detektion des Sekundenschlafs daraus
- Direkt auf den Fahrer gerichtete Kamera, wodurch der Sekundenschlaf des Fahrers durch direkte Videobeobachtung erkannt wird
- Remotesystem:
   ∘ Kontaktfreie Messung ohne Beeinträchtigung des Fahrers
   ∘ keine Anbringung am Kopf oder anderswo am Fahrer erforderlich
- Methodische Vorteile:
   ∘ Auswertung von Primärdaten, die videobasiert aus dem Gesicht des Fahrers abgeleitet werden
   ∘ Einsatz alternativer Algorithmen, die die beschriebene Gesamtfunktionalität (Erkennung von Sekundenschlaf/Mikroschlaf) oder einzelne Teilfunktionen (z. B. Gesichtserkennung, die Entfernung von Reflexen usw.) ersetzen

Anwendungsgebiete sind beispielsweise der Automotive Bereich sowie alle sonstigen Transportbereiche mittels Bus, Bahn, Schiff, U-Boot, LKW usw. oder alle sicherheitsrelevanten Bereiche, z. B. in Kraftwerken, für Fluglotsen, zur Verkehrsüberwachung usw. Die Vorrichtung kann in bestehende Fahrassistenzsysteme oder Überwachungssysteme integriert werden, sofern eine auf den Nutzer gerichtete Kamera zur Verfügung gestellt wird. Alternativ oder ergänzend kann ein Sekundenschlafwarner als eigenständiges System (abgeschlossene Hardware) verkauft werden. Dieses muss am Bestimmungsort so angebracht werden (im Normalfall einmalig), so dass der Nutzer von der Kamera erfasst wird. Die Anbringung kann von Fachpersonal durchgeführt werden, insbesondere, wenn noch eine Verbindung mit anderen Systemen hergestellt werden soll, z. B. mit dem CAN-Bus im PKW. Die bloße Anbringung und Ausrichtung auf den Nutzer kann von jedermann durchgeführt werden, beispielsweise per Saugnapfhalterung innen an der Windschutzscheibe eines PKW.

Ausführungsbeispielen zeigen, dass die individuell bei der beobachteten Person erkannte Dauer des Zwinkern, genutzt werden kann, um diese kurzen Lidschlusszeiten nicht schon als Sekundenschlaf zu bewerten. Mit "spontanem Lidschlag" bzw. "spontanem Lidschluss" ist in diesem Dokument beispielsweise ein Zwinkern gemeint.

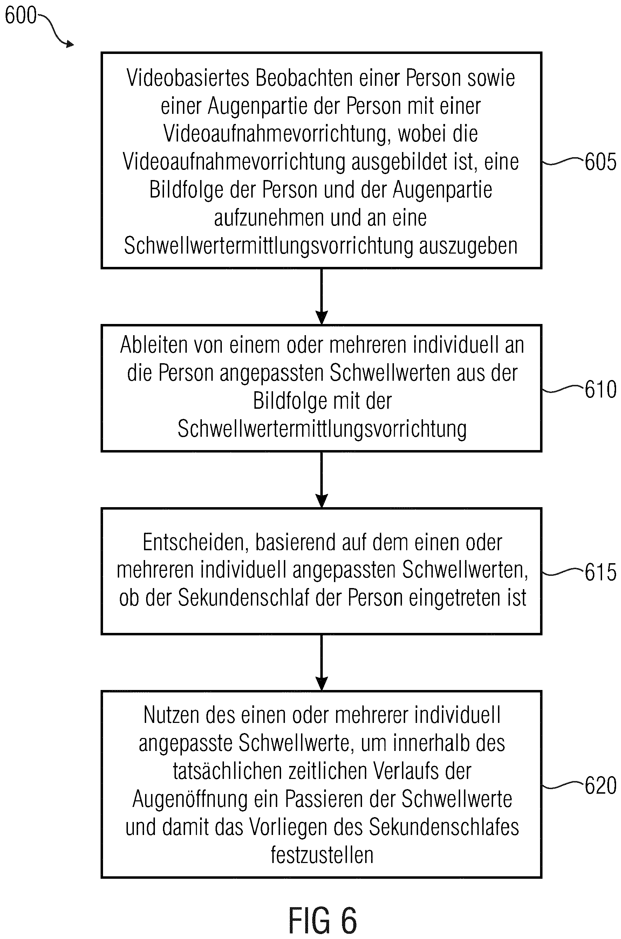

Weitere Ausführungsbeispiele zeigen ein Verfahren 600 zur Detektion eines Sekundenschlafs mit den Schritten 605 "Videobasiertes Beobachten einer Person sowie einer Augenpartie der Person mit einer Videoaufnahmevorrichtung, wobei die Videoaufnahmevorrichtung ausgebildet ist, eine Bildfolge der Person und der Augenpartie aufzunehmen und an eine Schwellwertermittlungsvorrichtung auszugeben", 610 "Ableiten von einem oder mehreren individuell an die Person angepassten Schwellwerten aus der Bildfolge mit der Schwellwertermittlungsvorrichtung", 615 "Entscheiden, basierend auf dem einen oder mehreren individuell angepassten Schwellwerten, ob der Sekundenschlaf der Person eingetreten ist" und 620 "Nutzen des einen oder mehrerer individuell angepasste Schwellwerte, um innerhalb des tatsächlichen zeitlichen Verlaufs der Augenöffnung ein Überschreiten bzw. Unterschreiten der Schwellwerte und damit das Vorliegen des Sekundenschlafes festzustellen". Ein schematisches Blockdiagramm des Verfahrens ist in Fig. 6 gezeigt.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder einer elektronischen Schaltung durchgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist. Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

### Quellen

[1] Timm, Barth (2011): Accurate Eye Centre Localisation by means of Gradients
[2] Viola and Jones, "Rapid object detection using a boosted cascade of simple features", Computer Vision and Pattern Recognition, 2001
[3] Tim F. Cootes, Chris J. Taylor: Active Shape Models - "Smart Snakes". In: David Hogg u.a. (Hrsg.): BMVC92. Proceedings of the British Machine Vision Conference; 22.-24. September 1992, Leeds. Springer-Verlag, Berlin 1992, ISBN 3-540-19777-X, S. 266-275.
[4] T.F. Cootes, G.J. Edwards, C.J. Taylor: "Active Appearance Models", in: IEEE Transactions on Pattern Analysis and Machine Intelligence, Vol. 23, No. 6, June 2001, pp. 681-685
[5] DeMenthon, Daniel F., and Larry S. Davis. "Model-based object pose in 25 lines of code." Computer Vision-ECCV'92. Springer Berlin Heidelberg, 1992.
[6] Zhi-Hua Zhou, Xin Geng: "Projection functions for eye detection." Pattern Recognition, Volume 37, Issue 5, May 2004, Pages 1049-1056

## Patentansprüche

1. Vorrichtung (5) zur Detektion eines Sekundenschlafs, mit folgenden Merkmalen:
einer Videoaufnahmevorrichtung (10) zum videobasierten Beobachten einer Person (15) sowie einer Augenpartie der Person (15), wobei die Videoaufnahmevorrichtung (10) ausgebildet ist, eine Bildfolge der Person (15) und der Augenpartie aufzunehmen und an eine Schwellwertermittlungsvorrichtung (20) auszugeben;
die Schwellwertermittlungsvorrichtung (20), die ausgebildet ist, um mehrere individuell an die Person (15) angepasste Schwellwerte (80, 85) aus der Bildfolge abzuleiten; und
einen Schwellwertauswerter (25), der ausgebildet ist, um basierend auf den mehreren individuell angepassten Schwellwerten (80, 85) zu entscheiden, ob der Sekundenschlaf der Person (15) eingetreten ist;
wobei der eine oder mehrere individuell angepasste Schwellwerte (80, 85) genutzt werden, um innerhalb des tatsächlichen zeitlichen Verlaufs der Augenöffnung ein Passieren der Schwellwerte (80, 85) und damit das Vorliegen des Sekundenschlafes festzustellen;
wobei ein erster individuell angepasster Schwellwert (80) den Übergang des geöffneten zum geschlossenen Auge kennzeichnet, wobei bei einem Passieren des ersten individuell angepassten Schwellwerts (80) ein Lidschluss vorliegt;
wobei sich ein zweiter individuell angepasster Schwellwert (85) auf eine zeitliche Dauer des Lidschlusses bezieht, wobei eine individuelle Lidschlusszeit bestimmt wird, um bei einem Überschreiten der individuellen Lidschlusszeit den Lidschluss als Sekundenschlaf zu bewerten.

2. Vorrichtung (5) gemäß Anspruch 1, wobei die individuell angepassten Schwellwerte in Kombination mit einem bestimmten Pupillenmittelpunkt zur Detektion des Sekundenschlafs verwendet werden.

3. Vorrichtung (5) gemäß einem der vorherigen Ansprüche, wobei der erste individuell angepasste Schwellwert (80) zwischen einem ersten individuell ermittelten Referenzwert (70), der ein geöffnetes Auge beschreibt und einem zweiten individuell ermittelten Referenzwert (75), der ein geschlossenes Auge beschreibt, liegt.

4. Vorrichtung gemäß einem der vorherigen Ansprüche, wobei die Schwellwertermittlungsvorrichtung (20) ausgebildet ist, die individuell ermittelten Referenzwerte (70, 75) und den einen oder mehrere individuell ermittelte Schwellwerte (80, 85) kontinuierlich durch die Auswertung der auftretenden Lidschläge während des Betriebs anzupassen.

5. Vorrichtung (5) gemäß Anspruch 4, wobei die Schwellwertermittlungsvorrichtung (20) ausgebildet ist, einen Lidschlag anhand eines Musters im zeitlichen Augenöffnungsverlauf zu bestimmen, wobei das Muster, ausgehend vom ersten individuell ermittelten Referenzwert (70) ein Unterschreiten des ersten individuell angepassten Schwellwertes (80) und eine Rückkehr zum ersten Referenzwert (70) innerhalb einer vorgegebenen Zeit aufweist.

6. Vorrichtung (5) gemäß einem der vorherigen Ansprüche, wobei die Schwellwertermittlungsvorrichtung (20) ausgebildet ist, den einen oder mehrere individuell angepasste Schwellwerte (80, 85) an die Anatomie und Physiologie der Person (15) anzupassen.

7. Vorrichtung (5) gemäß einem der vorherigen Ansprüche, wobei der erste individuell angepasste Schwellwert (80) eine Hysterese (90a, 90b) aufweist, die ausgebildet ist, einen Lidschluss bei einem geringeren Augenöffnungswinkel beim Übergang vom geöffneten zum geschlossenen Auge und bei einem größeren Augenöffnungswinkel beim Übergang vom geschlossenen zum geöffneten Auge festzulegen.

8. Vorrichtung (5) gemäß einem der vorherigen Ansprüche, wobei die Schwellwertermittlungsvorrichtung (20) eine Ermittlung einer Kopfpose umfasst, wobei die Schwellwertermittlungsvorrichtung (20) ausgebildet ist, den einen oder mehrere individuell angepasste Schwellwerte (80, 85) anhand der ermittelten Kopfpose anzupassen.

9. Vorrichtung (5) gemäß Anspruch 8, wobei der Schwellwertauswerter (25) ausgebildet ist, abhängig von der Kopfpose das Vorliegen des Sekundenschlafes anhand eines Auges oder anhand beider Augen der Person (15) zu bestimmen.

10. Vorrichtung (5) gemäß einem der vorherigen Ansprüche, wobei ein zweiter individuell angepasster Schwellwert (85) an die aktuelle Geschwindigkeit, mit der sich die Person (15) fortbewegt, angepasst ist.

11. Vorrichtung (5) gemäß einem der vorherigen Ansprüche, wobei die Vorrichtung (5) einen GPS-Empfänger (45) und/oder einen Beschleunigungssensor (50) aufweist, der ausgebildet ist, eine Fortbewegungsgeschwindigkeit der Vorrichtung (5) zu erfassen, wobei ausgehend von der Fortbewegungsgeschwindigkeit auf eine Geschwindigkeit der Person (15) und/oder eines Fahrzeugs geschlossen wird.

12. Vorrichtung (5) gemäß einem der vorherigen Ansprüche, wobei die Vorrichtung (5) eine Beleuchtungsquelle (55) aufweist, die Strahlung oberhalb eines für die Person (15) sichtbaren Wellenlängenbereichs aussendet, wobei die Videoaufnahmevorrichtung (10) ausgebildet ist, die Strahlung zu detektieren.

13. Vorrichtung (5) gemäß Anspruch 12, wobei die Vorrichtung (5) eine weitere Beleuchtungsquelle (60) aufweist, die von der Beleuchtungsquelle (55) entfernt angeordnet ist;
wobei die Videoaufnahmevorrichtung (10) ausgebildet ist, eine Kombination von Bildaufnahmen, bei denselben die Person (15) durch aufeinanderfolgendes Ausleuchten der Augenpartie mit der Beleuchtungsquelle (55) und der weiteren Beleuchtungsquelle (60) beleuchtet ist, zu bestimmen, um Reflexe im Bereich der Augenpartie der Person (15) zu vermeiden.

14. Verfahren (600) zur Detektion eines Sekundenschlafs, mit folgenden Schritten:
Videobasiertes Beobachten einer Person (15) sowie einer Augenpartie der Person (15) mit einer Videoaufnahmevorrichtung (10), wobei die Videoaufnahmevorrichtung (10) ausgebildet ist, eine Bildfolge der Person (15) und der Augenpartie aufzunehmen und an eine Schwellwertermittlungsvorrichtung (20) auszugeben;
Ableiten von einem oder mehreren individuell an die Person (15) angepassten Schwellwerten aus der Bildfolge mit der Schwellwertermittlungsvorrichtung (20);
Entscheiden, basierend auf dem einen oder mehreren individuell angepassten Schwellwerten (80, 85) mit einem Schwellwertauswerter (25), ob der Sekundenschlaf der Person (15) eingetreten ist; und
Nutzen des einen oder mehrerer individuell angepasster Schwellwerte (80, 85), um innerhalb des tatsächlichen zeitlichen Verlaufs der Augenöffnung ein Passieren der Schwellwerte (80, 85) und damit das Vorliegen des Sekundenschlafes festzustellen;
wobei ein erster individuell angepasster Schwellwert (80) den Übergang des geöffneten zum geschlossenen Auge kennzeichnet, wobei bei einem Unterschreiten des ersten individuell angepassten Schwellwerts (80) ein Lidschluss vorliegt;
wobei sich ein zweiter individuell angepasster Schwellwert (85) auf eine zeitliche Dauer des Lidschlusses bezieht, wobei eine individuelle Lidschlusszeit bestimmt wird, um bei einem Überschreiten der individuellen Lidschlusszeit den Lidschluss als Sekundenschlaf zu bewerten.

15. Computerprogramm zur Detektion eines Sekundenschlafs gemäß des Verfahrens aus Anspruch 14.

## Claims

1. Device (5) for detecting micro-sleep, comprising:
a video recording device (10) for video-based monitoring of a person (15) and of an eye area of said person (15), the video recording device (10) being configured to record a sequence of pictures of the person (15) and of the eye area and to output same to a threshold determination device (20);
the threshold determination device (20) configured to derive multiple thresholds (80, 85) individually adapted to the person (15) from the sequence of pictures; and
a threshold evaluator (25) configured to decide, on the basis of the multiple individually adapted thresholds (80, 85), whether or not the person (15) is experiencing micro-sleep;
the one or more individually adapted thresholds (80, 85) being utilized for establishing whether or not the thresholds (80, 85) have been passed within the actual time curve of the eye opening and, thus, whether or not the person is experiencing micro-sleep;
a first individually adapted threshold (80) characterizing the transition from the opened to the closed eye, an eyelid closure being established to have occurred if the first individually adapted threshold (80) has been passed;
a second individually adapted threshold (85) referring to a duration of the eyelid closure, an individual eyelid closure time being determined so as to evaluate the eyelid closure as being an indication of micro-sleep if the individual eyelid closure time is exceeded.

2. Device (5) as claimed in claim 1, wherein the individually adapted thresholds are utilized in combination with a specific pupil center for detecting micro-sleep.

3. Device (5) as claimed in any of the previous claims, wherein the first individually adapted threshold (80) lying between a first individually determined reference value (70) describing an opened eye and a second individually determined reference value (75) describing a closed eye.

4. Device as claimed in any of the previous claims, wherein the threshold determination device (20) is configured to continually adapt the individually determined reference values (70, 75) and the one or more individually determined thresholds (80, 85) on the fly by evaluating the blinks that occur.

5. Device (5) as claimed in claim 4, wherein the threshold determination device (20) is configured to determine a blink by means of a pattern in the time curve of the eye opening, the pattern comprising, starting from the first individually determined reference value (70), falling below the first individually adapted threshold (80) and a return to the first reference value (70) within a predefined time period.

6. Device (5) as claimed in any of the previous claims, wherein the threshold determination device (20) is configured to adapt the one or more individually adapted thresholds (80, 85) to the anatomy and physiology of the person (15).

7. Device (5) as claimed in any of the previous claims, wherein the first individually adapted threshold (80) comprises a hysteresis (90a, 90b) configured to establish an eyelid closure at a relatively small eye opening angle during transition from the opened to the closed eye and at a relatively large eye opening angle during transition from the closed to the opened eye.

8. Device (5) as claimed in any of the previous claims, wherein the threshold determination device (20) includes determining a head pose, the threshold determination device (20) being configured to adapt the one or more individually adapted thresholds (80, 85) by means of the head pose determined.

9. Device (5) as claimed in claim 8, wherein the threshold evaluator (25) is configured to determine whether or not the person is experiencing micro-sleep by means of one eye or by means of both eyes of the person (15) as a function of the head pose.

10. Device (5) as claimed in any of the previous claims, wherein a second individually adapted threshold (85) is adapted to the current speed at which the person (15) is travelling.

11. Device (5) as claimed in any of the previous claims, the device (5) comprising a GPS receiver (45) and/or an acceleration sensor (50) configured to capture a speed of travel of the device (5), conclusions being drawn from said speed of travel as to a speed of the person (15) and/or of a vehicle.

12. Device (5) as claimed in any of the previous claims, the device (5) comprising a source of illumination (55) emitting radiation above a wavelength range visible to the person (15), the video recording device (10) being configured to detect the radiation.

13. Device (5) as claimed in claim 12, the device (5) comprising a further source of illumination (60) arranged at a distance from the source of illumination (55);
wherein the video recording device (10) is configured to determine a combination of pictures taken in which the person (15) is illuminated by subsequent illumination of the eye area by the source of illumination (55) and the further source of illumination (60) so as to avoid any reflections in the region of the eye area of the person (15).

14. Method (600) of detecting micro-sleep, comprising:
video-based monitoring of a person (15) and of an eye area of said person (15) by using a video recording device (10), the video recording device (10) being configured to record a sequence of pictures of the person (15) and of the eye area and to output same to a threshold determination device (20);
deriving one or more thresholds individually adapted to the person (15) from the sequence of pictures by using the threshold determination device (20);
deciding, on the basis of the one or more individually adapted thresholds (80, 85), by using a threshold evaluator (25), whether or not the person (15) is experiencing micro-sleep; and
utilizing the one or more individually adapted thresholds (80, 85) so as to establish whether or not the thresholds (80, 85) have been passed within the actual time curve of the eye opening and, thus, whether or not the person is experiencing micro-sleep;
a first individually adapted threshold (80) characterizing the transition from the opened to the closed eye, an eyelid closure being established to have occurred if the first individually adapted threshold (80) is fallen below;
a second individually adapted threshold (85) referring to a duration of the eyelid closure, an individual eyelid closure time being determined so as to evaluate the eyelid closure as being an indication of micro-sleep if the individual eyelid closure time is exceeded.

15. Computer program for detecting micro-sleep in accordance with the method as claimed in claim 14.

## Revendications

1. Dispositif (5) permettant de détecter un micro-sommeil, aux caractéristiques suivantes:
un dispositif d'enregistrement vidéo (10) pour l'observation à base de vidéo d'une personne (15) ainsi que d'une partie de l'oeil de la personne (15), où le dispositif d'enregistrement vidéo (10) est conçu pour enregistrer une séquence d'images de la personne (15) et la partie de l'oeil et la sortir vers un dispositif de détermination de valeur de seuil (20);
le dispositif de détermination de valeur de seuil (20) qui est conçu pour dériver de la séquence d'images plusieurs valeurs de seuil (80, 85) adaptées individuellement à la personne (15); et
un évaluateur de valeur de seuil (25) qui est conçu pour décider, sur base des plusieurs valeurs de seuil adaptées individuellement (80, 85), s'il s'est produit le micro-sommeil de la personne (15);
dans lequel les une ou plusieurs valeurs de seuil adaptées individuellement (80, 85) sont utilisées pour constater pendant le déroulement dans le temps effectif de l'ouverture de l'oeil un passage des valeurs de seuil (80, 85) et, de ce fait, la présence du micro-sommeil;
dans lequel une première valeur de seuil adaptée individuellement (80) caractérise la transition de l'oeil ouvert à l'oeil fermé, dans lequel est présente, lors d'un passage de la première valeur de seuil adaptée individuellement (80), une fermeture de paupière;
dans lequel une deuxième valeur de seuil adaptée individuellement (85) se réfère à une durée de la fermeture de paupière, où une durée de fermeture de paupière individuelle est déterminée pour évaluer, en cas de dépassement de la durée de fermeture de paupière individuelle, la fermeture de paupière comme micro-sommeil.

2. Dispositif (5) selon la revendication 1, dans lequel les valeurs de seuil adaptées individuellement sont utilisées en combinaison avec un centre de pupille déterminé pour détecter le micro-sommeil.

3. Dispositif (5) selon l'une des revendications précédentes, dans lequel la première valeur de seuil adaptée individuellement (80) se situe entre une première valeur de référence déterminée individuellement (70) qui décrit un oeil ouvert et une deuxième valeur de référence déterminée individuellement (75) qui décrit un oeil fermé.

4. Dispositif selon l'une des revendications précédentes, dans lequel le dispositif de détermination de valeur de seuil (20) est conçu pour adapter en continu les valeurs de référence déterminées individuellement (70, 75) et les une ou plusieurs valeurs de seuil déterminées individuellement (80, 85) par l'évaluation des battements de cils pendant le fonctionnement.

5. Dispositif (5) selon la revendication 4, dans lequel le dispositif de détermination de valeur de seuil (20) est conçu pour déterminer un battement de cils à l'aide d'un modèle pendant le déroulement dans le temps de l'ouverture de l'oeil, où le modèle présente, partant de la première valeur de référence (70) déterminée individuellement, une descente au-dessous de la première valeur de seuil adaptée individuellement (80) et un retour à la première valeur de référence (70) dans un laps de temps prédéterminé.

6. Dispositif (5) selon l'une des revendications précédentes, dans lequel le dispositif de détermination de valeur de seuil (20) est conçu pour adapter les une ou plusieurs valeurs de seuil adaptées individuellement (80, 85) à l'anatomie et à la Physiologie de la personne (15).

7. Dispositif (5) selon l'une des revendications précédentes, dans lequel la première valeur de seuil adaptée individuellement (80) présente une hystérésis (90a, 90b) qui est conçue pour constater une fermeture de paupière à un petit angle d'ouverture de l'oeil lors du passage de l'oeil ouvert à l'oeil fermé, et à un grand angle d'ouverture de l'oeil lors du passage de l'oeil fermé à l'oeil ouvert.

8. Dispositif (5) selon l'une des revendications précédentes, dans lequel le dispositif de détermination de valeur de seuil (20) comprend une détermination d'une pose de tête, dans lequel le dispositif de détermination de valeur de seuil (20) est conçu pour adapter les une ou plusieurs valeurs de seuil adaptées individuellement (80, 85) à l'aide de la pose de tête déterminée.

9. Dispositif (5) selon la revendication 8, dans lequel l'évaluateur de valeur de seuil (25) est conçu pour déterminer, en fonction de la pose de tête, la présence du micro-sommeil à l'aide d'un oeil ou à l'aide des deux yeux de la personne (15).

10. Dispositif (5) selon l'une des revendications précédentes, dans lequel une deuxième valeur de seuil (85) adaptée individuellement est adaptée à la vitesse actuelle à laquelle se déplace la personne (15).

11. Dispositif (5) selon l'une des revendications précédentes, dans lequel le dispositif (5) présente un récepteur GPS (45) et/ou un capteur d'accélération (50) qui est conçu pour détecter une vitesse de déplacement du dispositif (5), dans lequel il est conclu, partant de la vitesse de déplacement, à une vitesse de la personne (15) et/ou d'un véhicule.

12. Dispositif (5) selon l'une des revendications précédentes, dans lequel le dispositif (5) présente une source lumineuse (55) qui émet un rayonnement au-dessus d'une plage de longueurs d'onde visible pour la personne (15), dans lequel le dispositif d'enregistrement vidéo (10) est conçu pour détecter le rayonnement.

13. Dispositif (5) selon la revendication 12, dans lequel le dispositif (5) présente une autre source lumineuse (60) qui est disposée éloignée de la source lumineuse (55);
dans lequel le dispositif d'enregistrement vidéo (10) est conçu pour déterminer une combinaison de prises d'image lors desquelles la personne (15) est éclairée par illumination successive de la partie de l'oeil par la source lumineuse (55) et l'autre source lumineuse (60), pour éviter des reflets à l'endroit de la partie de l'oeil de la personne (15).

14. Procédé (600) permettant de détecter un micro-sommeil, aux étapes suivantes consistant à:
observer à base de vidéo une personne (15) ainsi qu'une partie de l'oeil de la personne (15) par un dispositif d'enregistrement vidéo (10), où le dispositif d'enregistrement vidéo (10) est conçu pour enregistrer une séquence d'images de la personne (15) et de la partie de l'oeil et la sortir vers un dispositif de détection de valeur de seuil (20);
dériver de la séquence d'images une ou plusieurs valeurs de seuil adaptées individuellement à la personne (15), par le dispositif de détermination de valeur de seuil (20);
décider, sur base des une ou plusieurs valeurs de seuil adaptées individuellement (80, 85), par un évaluateur de valeur de seuil (25), s'il s'est produit le micro-sommeil de la personne (15); et
utiliser les une ou plusieurs valeurs de seuil adaptées individuellement (80, 85) pour constater pendant le déroulement dans le temps effectif de l'ouverture de l'oeil un passage des valeurs de seuil (80, 85) et, de ce fait, la présente du micro-sommeil;
dans lequel une première valeur de seuil adaptée individuellement (80) caractérise la transition de l'oeil ouvert à l'oeil fermé, dans lequel est présente, lors d'une descente au-dessous de la première valeur de seuil adaptée individuellement (80), une fermeture de paupière;
dans lequel une deuxième valeur de seuil adaptée individuellement (85) se réfère à une durée de la fermeture de paupière, où une durée de fermeture de paupière individuelle est déterminée pour évaluer, en cas de dépassement de la durée de fermeture de paupière individuelle, la fermeture de paupière comme micro-sommeil.

15. Programme d'ordinateur pour détecter un micro-sommeil selon le procédé de la revendication 14.
